# EUROPÄISCHE PATENTSCHRIFT

(11) **EP 1 928 811 B1**
(45) Veröffentlichungstag und Bekanntmachung des Hinweises auf die Patenterteilung: **31.12.2008**
(21) Anmeldenummer: 06793110.5
(22) Anmeldetag: 31.08.2006
(51) Int. Cl.: C07C 17/16, C07C 19/043, C07B 39/00

(54) **VERFAHREN ZUR CHLORIERUNG VON ALKOHOLEN**
METHOD FOR CHLORINATING ALCOHOLS
PROCEDE DE CHLORATION D'ALCOOLS

(30) Priorität: 10.09.2005 DE 102005043141
(43) Veröffentlichungstag der Anmeldung: 11.06.2008
(73) Patentinhaber: BASF SE, 67056 Ludwigshafen (DE)
(72) Erfinder: ROHDE, Thorsten, 68305 Mannheim (DE); HUTTENLOCH, Oliver, 75228 Ispringen (DE); OSSWALD, Friederike, 68163 Mannheim (DE); WISSEL, Kathrin, 64625 Bensheim (DE)
(86) Internationale Anmeldenummer: PCT/EP2006/065873
(87) Internationale Veröffentlichungsnummer: WO 2007/028761

(56) Entgegenhaltungen:
- EP-A2- 0 223 421

## Beschreibung

Die vorliegende Erfindung betrifft ein Verfahren zur Herstellung von organischen Chloriden, bei denen das Chloratom an eine CH₂-Gruppe gebunden ist, durch Umsetzung der entsprechenden Alkohole mit Thionylchlorid in Gegenwart eines Triarylphosphinoxids bei einer Temperatur von 20 bis 200°C und einem Druck von 0,01 bis 10 MPa abs.

Alkylchloride sind wichtige Zwischenprodukte bei der Synthese chemischer Produkte, wie beispielsweise von Farbstoffen, Pharma- und Agrarwirkstoffen, Galvanohilfsmitteln, Liganden für Homogenkatalysatoren, Desinfektionsmitteln, Steroiden und Wuchshormonen.

Die Chlorierung von Alkoholen mit Chlorierungsmitteln, wie beispielsweise Thionylchlorid, Phosphortrichlorid oder Phosgen ist allgemein bekannt. Vorzugsweise werden dafür sogenannte Chlorierungskatalysatoren eingesetzt.

US 2,331,681 beschreibt die Chlorierung von Glycolnitril zu Chloroacetonitril mit Thionylchlorid in Gegenwart der organischen Basen Pyridin, Dimethylanilin sowie Chinolin. Ungünstig ist, dass hierfür äquimolare Mengen an Base benötigt werden und diese nach der Umsetzung abgetrennt und entsorgt werden muss.

EP-A 0 645 357 lehrt ein Verfahren zur Herstellung von Alkylchloriden aus dem entsprechenden Alkohol und einer stöchiometrischen Mengen eines Katalysator-Addukts ("Vilsmeier-Salz") aus einem N,N-Dialkylformamid und Phosgen oder Thionylchlorid. Nachteilig an diesem Verfahren ist der Einsatz äquimolarer Mengen an Katalysator und die sukzessive Zuführung von Alkohol und des Chlorierungsmittels.

GB-A 2,182,039 offenbart die Chlorierung von Alkoholen mit Thionylchlorid oder Phosgen in Gegenwart von Triphenylphosphinoxid oder Triphenylphosphinsulfid. In den Beispielen VIII und IX ist die Chlorierung von 2,3,6,3',4'-Pentaacetyl-saccharose mit Thionylchlorid in Gegenwart von Triphenylphosphinoxid und Toluol beziehungsweise 1,2-Dichlorethan beschrieben. Aus Beispiel VIII errechnet sich ein molares Verhältnis des eingesetzten Triphenylphosphinoxids zur Stoffmenge der zu chlorierenden OH-Gruppen von etwa 0,7 und aus Beispiel IX von etwa 1,7.

EP-A-0 223 421 offenbart die Chlorierung von Alkoholen in Gegewwart von Triphenylphosphin.

DE-A 41 16 365 lehrt die Herstellung von Alkyl-, Alkenyl- und Alkinylchloriden durch Umsetzung der entsprechenden Alkohole mit Phosgen oder Thionylchlorid in Gegenwart eines aliphatischen, cycloaliphatischen oder cyclisch-aliphatischen Phosphinoxids als Katalysator. Dabei wird auf Seite 2, Zeile 10 bis 12 hervorgehoben, dass gemäß der zuvor zitierten GB-A 2,182,039-Schrift Triarylphosphinoxide wegen ihrer geringen Reaktivität in überstöchiometrischen Mengen eingesetzt werden müssen und aufgrund dieser hohen Mengen und der geringen Löslichkeit die Aufarbeitung des Reaktionsgemisches erschweren. Nachteilig am Einsatz aliphatischer, cycloaliphatischer und cyclisch-aliphatischer Phosphinoxide ist jedoch deren - insbesondere gegenüber Triphenylphosphinoxid - schlechtere Verfügbarkeit infolge aufwändiger Herstellungsverfahren, was sich wirtschaftlich gesehen auch im Preis niederschlägt.

Aufgabe der vorliegenden Erfindung war es, ein Verfahren zur Herstellung von organischen Chloriden, bei denen das Chloratom an eine CH₂-Gruppe gebunden ist, zu finden, welches die Nachteile des Standes der Technik nicht besitzt, leicht zugängliche und technisch gut verfügbare Edukte sowie gegebenenfalls nur leicht zugängliche und technisch gut verfügbare Hilfsstoffe/Katalysatoren erfordert, unter milden Temperaturen und Drücken zu einem hohen Umsatz, einer hohen Selektivität und einer hohen Raum/Zeit-Ausbeute an Wertprodukt führt, eine einfache Aufarbeitung des Reaktionsgemisches ermöglicht und bei dem das Wertprodukt in hoher Reinheit gewonnen werden kann.

Demgemäß wurde ein Verfahren zur Herstellung von organischen Chloriden, bei denen das Chloratom an eine CH₂-Gruppe gebunden ist, durch Umsetzung der entsprechenden Alkohole mit Thionylchlorid in Gegenwart eines Triarylphosphinoxids bei einer Temperatur von 20 bis 200°C und einem Druck von 0,01 bis 10 MPa abs gefunden, das dadurch gekennzeichnet ist, dass man das Triarylphosphinoxid in einem molaren Verhältnis zur Stoffmenge der zu chlorierenden OH-Gruppen von 0,0001 bis 0,5 einsetzt.

Aufgrund der technischen Lehren von DE-A 41 16 365 und GB-A 2,182,039, nach denen Triarylphosphinoxide wegen ihrer geringen Reaktivität in überstöchiometrischen Mengen eingesetzt werden müssen, war es völlig überraschend, dass zur Herstellung von organischen Chloriden, bei denen das Chloratom an eine CH₂-Gruppe gebunden ist, durch Umsetzung der entsprechenden Alkohole mit Thionylchlorid gerade unterstöchiometrische Mengen an Triarylphosphinoxid absolut ausreichend sind.

Beim erfindungsgemäßen Verfahren setzt man das Triarylphosphinoxid bevorzugt in einem molaren Verhältnis zur Stoffmenge der zu chlorierenden OH-Gruppen von 0,001 bis 0,5, besonders bevorzugt von 0,001 bis 0,1 und ganz besonders bevorzugt von 0,005 bis 0,05 ein.

Beim erfindungsgemäßen Verfahren setzt man im Allgemeinen als Alkohol eine Verbindung der allgemeinen Formel (I) 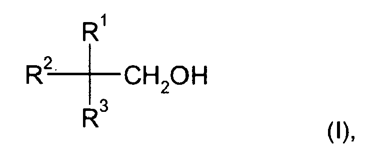 in der die Reste R¹ bis R³ unabhängig voneinander
- für Wasserstoff oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen;
- zwei Reste zusammen für einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 40 Kohlenstoffatomen; oder
- alle drei Reste zusammen für einen dreibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 50 Kohlenstoffatomen
stehen, ein.

Als Heteroatome kommen prinzipiell alle Heteroatome in Frage, welche in der Lage sind, formell eine -CH₂-, eine -CH=, eine -C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-, -NR'-, -N=, -PR'-, -PR'₂ und -SiR'₂- genannt, wobei es sich bei den Resten R' um den verbleibenden Teil des Kohlenstoff enthaltenden Rests handelt.

Als funktionelle Gruppen kommen prinzipiell alle funktionellen Gruppen in Frage, welche an ein Kohlenstoffatom oder ein Heteroatom gebunden sein können. Als geeignete Beispiele seien -OH (Hydroxy), -Hal (Halogen), =O (insbesondere als Carbonylgruppe), -NH₂ (Amino), =NH (Imino), -COOH (Carboxy), -CONH₂ (Carboxamid), -SO₃H (Sulfo) und -CN (Cyano) genannt. Fuktionelle Gruppen und Heteroatome können auch direkt benachbart sein, so dass auch Kombinationen, wie etwa -COO- (Ester), -CONH-(sekundäres Amid) oder -CONR'- (tertiäres Amid), mit umfasst sind. Als Halogene (Hal) seien Fluor, Chlor, Brom und lod genannt.

Bevorzugt stehen die Reste R¹ bis R³ unabhängig voneinander für
- Wasserstoff;
- gegebenenfalls durch funktionelle Gruppen, Cycloalkyl, Aryl, Aryloxy und/oder Alkyloxy substituiertes und/oder durch ein oder mehrere Sauerstoffatome unterbrochenes C₁- bis C₂₀-Alkyl;
- gegebenenfalls durch funktionelle Gruppen, Cycloalkyl, Aryl, Aryloxy und/oder Alkyloxy substituiertes C₅- bis C₁₂-Cycloalkyl;
- gegebenenfalls durch funktionelle Gruppen, Cycloalkyl, Aryl, Aryloxy und/oder Alkyloxy substituiertes und/oder durch ein oder mehrere Sauerstoffatome unterbrochenes C₂- bis C₂₀-Alkenyl, wobei dieses eine oder mehrere C=C-Doppelbindungen enthalten kann;
- gegebenenfalls durch funktionelle Gruppen, Cycloalkyl, Aryl, Aryloxy und/oder Alkyloxy substituiertes C₅- bis C₁₂-Cycloalkenyl;
- gegebenenfalls durch funktionelle Gruppen, Cycloalkyl, Aryl, Aryloxy und/oder Alkyloxy substituiertes und/oder durch ein oder mehrere Sauerstoffatome unterbrochenes C₂- bis C₂₀-Alkinyl, wobei dieses eine oder mehrere C≡C-Dreifachbindungen enthalten kann; oder
- gegebenenfalls durch funktionelle Gruppen, Cycloalkyl, Aryl, Alkyl, Aryloxy und/oder Alkyloxy substituiertes C₆- bis C₁₂-Aryl;
oder zwei Reste zusammen für
- gegebenenfalls durch funktionelle Gruppen, Cycloalkyl, Aryl, Aryloxy und/oder Alkyloxy substituiertes und/oder durch ein oder mehrere Sauerstoffatome unterbrochenes C₄- bis C₃₀-Alkylen; oder
- gegebenenfalls durch funktionelle Gruppen, Cycloalkyl, Aryl, Aryloxy und/oder Alkyloxy substituiertes und/oder durch ein oder mehrere Sauerstoffatome unterbrochenes C₅- bis C₃₀-Alkenylen, wobei dieses eine oder mehrere C=C-Doppelbindungen enthalten kann;
oder alle drei Reste zusammen für
- einen dreibindigen, gegebenenfalls durch funktionelle Gruppen, Cycloalkyl, Aryl, Aryloxy und/oder Alkyloxy substituiertes und/oder durch ein oder mehrere Sauerstoffatome unterbrochenen, gesättigten oder ungesättigten C₆- bis C₄₀-Kohlenwasserstoffrest.

Bei gegebenenfalls durch funktionelle Gruppen, Cycloalkyl, Aryl, Aryloxy und/oder Alkyloxy substituiertes und/oder durch ein oder mehrere Sauerstoffatome unterbrochenes C₁- bis C₂₀-Alkyl handelt es sich beispielsweise um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, 2-Etylhexyl, 2,4,4-Trimethylpentyl, 1,1,3,3-Tetramethylbutyl, 1-Nonyl, 1-Decyl, 1-Undecyl, 1-Dodecyl, 1-Tridecyl, 1-Tetradecyl, 1-Pentadecyl, 1-Hexadecyl, 1-Heptadecyl, 1-Octadecyl, Nonadecyl, Eicosyl, Cyclopentylmethyl, 2-Cyclopentylethyl, 3-Cyclopentylpropyl, Cyclohexylmethyl, 2-Cyclohexylethyl, 3-Cyclohexylpropyl, Benzyl (Phenylmethyl), Diphenylmethyl (Benzhydryl), Triphenylmethyl, 1-Phenylethyl, 2-Phenylethyl, 3-Phenylpropyl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, Methoxy, Ethoxy, 1,3-Dioxolan-2-yl, 1,3-Dioxan-2-yl, 2-Methyl-1,3-dioxolan-2-yl, 4-Methyl-1,3-dioxolan-2-yl, Hydroxymethyl, 2-Hydroxyethyl, 2-Hydroxypropyl, 3-Hydroxypropyl, 4-Hydroxybutyl, 6-Hydroxyhexyl, 2-Hydroxymethyl-2-propyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, Acetyl, Chlormethyl, 2-Chlorethyl, Trichlormethyl, 1,1-Dimethyl-2-chlorethyl, Methoxymethyl, 2-Butoxyethyl, Diethoxymethyl, Diethoxyethyl, 2-Isopropoxyethyl, 2-Butoxypropyl, 2-Octyloxyethyl, 2-Methoxyisopropyl, 2-(Methoxycarbonyl)-ethyl, 2-(Ethoxycarbonyl)-ethyl, 2-(n-Butoxycarbonyl)-ethyl, 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-dioxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-dioxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bei gegebenenfalls durch funktionelle Gruppen, Cycloalkyl, Aryl, Aryloxy und/oder Alkyloxy substituiertes C₅- bis C₁₂-Cycloalkyl handelt es sich beispielsweise um Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Chlorcyclohexyl, Dichlorcyclohexyl oder Dichlorcyclopentyl.

Bei gegebenenfalls durch funktionelle Gruppen, Cycloalkyl, Aryl, Aryloxy und/oder Alkyloxy substituiertes und/oder durch ein oder mehrere Sauerstoffatome unterbrochenes C₂- bis C₂₀-Alkenyl handelt es sich beispielsweise um Vinyl, 2-Propenyl, 3-Butenyl, cis-2-Butenyl oder trans-2-Butenyl.

Bei gegebenenfalls durch funktionelle Gruppen, Cycloalkyl, Aryl, Aryloxy und/oder Alkyloxy substituiertes C₅- bis C₁₂-Cycloalkenyl handelt es sich beispielsweise um 3-Cyclopentenyl, 2-Cyclohexenyl, 3-Cyclohexenyl oder 2,5-Cyclohexadienyl.

Bei gegebenenfalls durch funktionelle Gruppen, Cycloalkyl, Aryl, Aryloxy und/oder Alkyloxy substituiertes und/oder durch ein oder mehrere Sauerstoffatome unterbrochenes C₂- bis C₂₀-Alkinyl handelt es sich beispielsweise um Ethinyl, 1-Propinyl oder 2-Propinyl.

Bei gegebenenfalls durch funktionelle Gruppen, Cycloalkyl, Aryl, Alkyl, Aryloxy und/oder Alkyloxy substituiertes C₆- bis C₁₂-Aryl handelt es sich beispielsweise um Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2-Nitrophenyl, 4-Nitrophenyl, 2,4-Dinitrophenyl, 2,6-Dinitrophenyl, 4-Dimethylaminophenyl, Methoxyethylphenyl oder Ethoxymethylphenyl.

Bilden zwei benachbarte Reste gemeinsam einen gegebenenfalls durch funktionelle Gruppen, Cycloalkyl, Aryl, Aryloxy und/oder Alkyloxy substituiertes und/oder durch ein oder mehrere Sauerstoffatome unterbrochenen C₄- bis C₃₀-Alkylen- oder C₅- bis C₃₀-Alkenylen-Rest, so handelt es sich beispielsweise um 1,3-Propylen, 1,4-Butylen, 1,5-Pentylen, 2-Oxa-1,3-propylen, 1-Oxa-1,3-propylen, 2-Oxa-1,3-propylen, 1-Oxa-1,3-propenylen oder 3-Oxa-1,5-pentylen.

Bilden alle drei Reste zusammen einen dreibindigen, gegebenenfalls durch funktionelle Gruppen, Cycloalkyl, Aryl, Aryloxy und/oder Alkyloxy substituiertes und/oder durch ein oder mehrere Sauerstoffatome unterbrochenen, gesättigten oder ungesättigten C₆- bis C₄₀-Kohlenwasserstoffrest, so handelt es sich beispielsweise um einen unsubsituierten oder substituierten Rest der allgemeinen Formel (II)

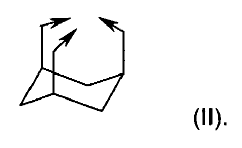

Enthalten die oben genannten Reste Heteroatome, so befinden sich zwischen zwei Heteroatomen in der Regel mindestens ein Kohlenstoffatom, bevorzugt mindestens zwei Kohlenstoffatome.

Bevorzugt setzt man beim erfindungsgemäßen Verfahren als Alkohole Methanol, Ethanol, 1-Propanol, 1-Butanol, 1-Pentanol, 1-Hexanol, 1-Heptanol, 1-Octanol, 1-Nonanol, 1-Decanol, Propargylalkohol, 2-Buten-1,4-diol, 2-Butin-1,4-diol, 1,2-Ethandiol, 1,2-Propandiol, 1,3-Propandiol, 1,2-Butandiol, 1,4-Butandiol, 1,5-Pentandiol, 1,6-Hexandiol, 1,2-Pentandiol, 1,2,4-Butantriol, Ethylenglycolmonomethylether, Ethylenglycolmonoethylether, Diethylenglycol, Diethylenglycolmonomethylether, Diethylenglycolmonoethylether, Triethylenglycol, Triethylenglycolmonomethylether, Triethylenglycolmonoethylether, Tetraethylenglycol, Tetraethylenglycolmonomethylether, Tetraethylenglycolmonoethylether, Pentaethylenglycol, Pentaethylenglycolmonomethylether, Pentaethylenglycolmonoethylether, Hexaethylenglycol, Hexaethylenglycolmonomethylether, Hexaethylenglycolmonoethylether, Butylenglycolmonomethylether, Butylenglycolmonoethylether, Butylenglycolmonobutylether, Dibutylenglycol, Dibutylenglycolmonomethylether, Dibutylenglycolmonoethylether, Dibutylenglycolmonobutylether, Tributylenglycol, Tributylenglycolmonomethylether, Tributylenglycolmonoethylether, Tributylenglycolmonobutylether, Tetraethylenglycol, Tetraethylenglycolmonomethylether, Tetraethylenglycolmonoethylether, Tetrabutylenglycolmonobutylether, Benzylalkohol, 2-Hydroxymethyl-benzylalkohol, 3-Hydroxymethyl-benzylalkohol, 4-Hydroxymethyl-benzylalkohol, 4-Methoxybenzylalkohol, 2,2-Dimethyl-propanol, 2-Ethylhexanol, 2-Ethylheptanol, 2-Propylheptanol, 2-Propylheptanol, Pentaerythritol, 2,2-Dimethyl-1,3-propandiol, 1,1,1-Tris(hydroxymethyl)ethan oder 1,1,1-Tris(hydroxymethyl)propan ein.

Besonders bevorzugt stehen die Reste R¹ bis R³ unabhängig voneinander für
- Wasserstoff;
- gegebenenfalls durch Hydroxy, Halogen, Cycloalkyl, Aryl, Aryloxy und/oder Alkyloxy substituiertes und/oder durch ein oder mehrere Sauerstoffatome unterbrochenes C₁- bis C₂₀-Alkyl;
- gegebenenfalls durch Hydroxy, Halogen, Cycloalkyl, Aryl, Aryloxy und/oder Alkyloxy substituiertes und/oder durch ein oder mehrere Sauerstoffatome unterbrochenes C₂- bis C₂₀-Alkenyl, wobei dieses eine oder mehrere C=C-Doppelbindungen enthalten kann;
- gegebenenfalls durch Hydroxy, Halogen, Cycloalkyl, Aryl, Aryloxy und/oder Alkyloxy substituiertes und/oder durch ein oder mehrere Sauerstoffatome unterbrochenes C₂- bis C₂₀-Alkinyl, wobei dieses eine oder mehrere C≡C-Dreifachbindungen enthalten kann; oder
- gegebenenfalls durch Hydroxy, Halogen, Cycloalkyl, Aryl, Alkyl, Aryloxy und/oder Alkyloxy substituiertes C₆- bis C₁₂-Aryl.

Ganz besonders bevorzugt setzt man als Alkohol eine Verbindung der allgemeinen Formel (I) ein, bei der maximal ein, und ganz stark bevorzugt kein Rest aus R¹ bis R³ Wasserstoff ist.

Insbesondere stehen die Reste R¹ bis R³ unabhängig voneinander für
- gegebenenfalls durch Hydroxy und/oder Halogen substituiertes und/oder durch ein oder mehrere Sauerstoffatome unterbrochenes C₁- bis C₂₀-Alkyl; oder
- gegebenenfalls durch Hydroxy und/oder Halogen substituiertes C₆- bis C₁₂-Aryl.

Ganz besonders bevorzugt setzt man beim erfindungsgemäßen Verfahren als Alkohole 1,6-Hexandiol, 2,2-Dimethyl-1,3-propandiol, 1,1,1-Tris(hydroxymethyl)ethan, 2-Ethylhexanol, Propargylalkohol und insbesondere 1,6-Hexandiol, 2,2-Dimethyl-1,3-propandiol und 1,1,1-Tris(hydroxymethyl)ethan ein.

Beim erfindungsgemäßen Verfahren können als Katalysator sowohl ein- oder mehrfach kernsubstituierte Triarylphosphinoxide als auch unsubstituiertes Triphenylphosphinoxid eingesetzt werden. Bevorzugt setzt man Triarylphosphinoxide der allgemeinen Formel (III) 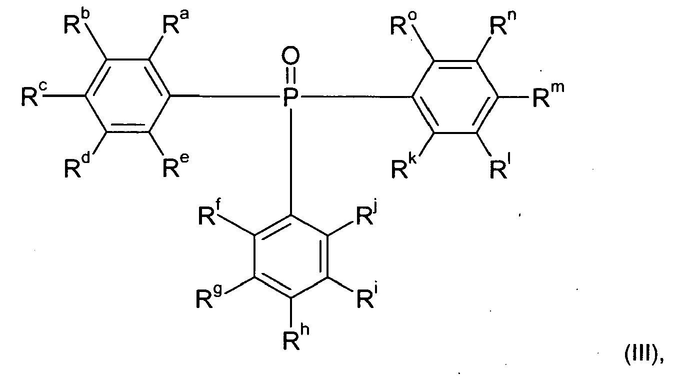 in der die Reste R^{a} bis R^{o} unabhängig voneinander für Wasserstoff oder C₁- bis C₆-Alkyl stehen, ein. Ganz besonders bevorzugt setzt man Triphenylphosphinoxid ein.

Die Zugabe des Alkohols, des Thionylchlorids, des Triarylphosphinoxids und eventuell einzusetzender Lösungsmittel kann auf unterschiedliche Art und Weise und in unterschiedlicher Reihenfolge erfolgen. Der Alkohol kann beispielsweise flüssig, fest oder gelöst in einem inerten Lösungsmittel eingesetzt werden. Ferner ist es möglich, einen Teil oder die gesamte Menge an Triarylphosphinoxid gelöst im oder vermischt mit dem Alkohol zuzugeben.

Die Zugabe von Thionylchlorid erfolgt in der Regel flüssig. Des weiteren ist es prinzipiell auch möglich, einen Teil oder die gesamte Menge an Triarylphosphinoxid im Thionylchlorid zu lösen und auf diese Art und Weise zuzugeben.

Alternativ zu den oben genannten Zugabearten für das Triarylphosphinoxid kann dieses auch separat, gegebenenfalls gelöst in inertem Lösungsmittel, zugegeben werden.

Im Allgemeinen ist es nicht erforderlich, ein Lösungsmittel zuzugeben, insbesondere dann nicht, wenn der eingesetzte Alkohol und/oder das durch die Umsetzung entstehende Reraktionsgemisch bei der gewählten Reaktionstemperatur flüssig vorliegen. Bei Reaktionsgemischen, welche bei der gewählten Reaktionstemperatur als Feststoff vorliegen würden, ist der Einsatz eines inerten Lösungsmittel jedoch in der Regel ratsam. Unter einem inerten Lösungsmittel sind Lösungsmittel zu verstehen, welche gegenüber dem eingesetzten Alkohol, dem Thionylchlorid, dem gebildeten organischen Chlorid und dem verwendeten Triarylphosphinoxid chemisch inert sind. "Chemisch inert" heißt, daß sich die Verdünnungsmittel unter den gewählten Bedingungen nicht mit den genannten Stoffen chemisch umsetzen. Als geeignete Lösungsmittel sind beispielsweise aromatische oder aliphatische Kohlenwasserstoffe genannt. Wird ein Lösungsmittel eingesetzt, so liegt bevorzugt dessen Siedepunkt vom Siedepunkt des gewünschten organischen Chlorids weit genug entfernt, um nachfolgend das Wertprodukt in einfacher Art und Weise und in der gewünschten Reinheit abtrennen zu können. Die Menge an eingesetzten Lösungsmittel liegt bevorzugt im Bereich von 100 bis 1000% der Menge, die erforderlich ist, um das Reaktionsgemisch in der Flüssigphase zu halten.

Bevorzugt legt man beim erfindungsgemäßen Verfahren den Alkohol und das Triarylphosphinoxid vor und bringt dieses auf die gewünschte Reaktionstemperatur. Anschließend beginnt man im Allgemeinen mit der Einleitung von Thionylchlorid, welches üblicherweise in flüssiger Form zugegeben wird.

Beim erfindungsgemäßen Verfahren setzt man das Thionylchlorid im Allgemeinen in einem molaren Verhältnis zur Stoffmenge der zu chlorierenden OH-Gruppen von 0,9 bis 5, bevorzugt von 0,95 bis 2, besonders bevorzugt von 0,99 bis 1,5 und ganz besonders bevorzugt von 1 bis 1,2 ein.

Die bei der Umsetzung freiwerdenden Reaktionsgase Chlorwasserstoff und Schwefeldioxid werden in der Regel kontinuierlich aus dem Reaktionsapparat abgeführt.

Das erfindungsgemäße Verfahren wird in der Regel halbkontinuierlich oder kontinuierlich betrieben. Bei der halbkontinuierlichen Fahrweise wird üblicherweise der Alkohol in einem geeigneten Reaktionsapparat vorgelegt und darin die Gesamtmenge an Triarylphosphinoxid oder zumindest ein Teil davon gelöst. Anschließend gibt man das Thionylchlorid, welches gegebenenfalls die restliche Menge des Triarylphosphinoxids enthält, entsprechend dem Reaktionsfortschritt kontinuierlich zu.

Bei der kontinuierlichen Fahrweise werden üblicherweise die Edukte und das Triarylphosphinoxid gleichzeitig einem geeigneten Reaktionsapparat zugeführt, wobei eine der zugeführten Menge entsprechende Menge gleichzeitig aus dem Reaktionsapparat abgeführt wird.

Als geeignete Reaktionsapparate seien prinzipiell alle Reaktionsappate genannt, welche für flüssig/flüssig-Reaktionen geeignet sind, wie beispielsweise Rührkessel.

Das erfindungsgemäße Verfahren führt man bei einem Druck von 0,01 bis 10 MPa abs, bevorzugt von 0,05 bis 5 MPa abs, besonders bevorzugt von 0,09 bis 0,5 MPa abs und ganz besonders bevorzugt von 0,09 bis 0,2 MPa abs durch.

Des Weiteren führt man das erfindungsgemäße Verfahren bei einer Temperatur von 20 bis 200°C, bevorzugt von 30 bis 180°C und besonders bevorzugt von 50 bis 160°C durch.

Nachdem die gewünschte Menge an Thionylchlorid zugegeben wurde, wird die erhaltene Reaktionslösung in der Regel noch für eine gewisse Zeit, im Allgemeinen 30 Minuten bis 6 Stunden unter den Reaktionsbedingungen zur Nachreaktion belassen. Um überschüssiges Thionylchlorid und dessen Reaktionsprodukte Chlorwasserstoff und Schwefeldioxid aus der Reaktionslösung zu entfernen beziehungsweise abzureichern, kann gegebenenfalls unter Durchmischung Inertgas durchgeleitet werden ("Strippen").

Der Reaktionsaustrag wird im Allgemeinen mit den bekannten Methoden aufgearbeitet. Vorzugsweise wird das gewünschte organische Chlorid durch fraktionierte Destillation isoliert. Das eingesetzte Triarylphosphinoxid kann dabei bei Bedarf durch Destillation zurückgewonnen und wieder eingesetzt werden.

In einer allgemeinen Ausführungsform zur halbkontinuierlichen Herstellung von organischen Chloriden, bei denen das Chloratom an eine CH₂-Gruppe gebunden ist, gibt man die gewünschte Menge des entsprechenden Alkohols und die gewünschte Menge an Triarylphosphinoxid in einen Rührkessel mit Rückflusskühler und bringt das Gemisch auf die gewünschte Reaktionstemperatur. Anschließend beginnt man mit der Zugabe von Thionylchlorid, wobei die Zugabegeschwindigkeit in der Regel derat eingestellt wird, dass das nicht umgesetzte Thionylchlorid schwach unter Rückfluss siedet. Nach Beendigung der Zugabe der gewünschten Menge an Thionylchlorid belässt man das Reaktionsgemisch unter weiterem Rühren für etwa 0,5 bis 6 Stunden zur Nachreaktion. Vorteilhafterweise strippt man anschließend restlichen Chlorwasserstoff und restliches Schwefeldioxid mit Stickstoff heraus. Zuletzt führt man das Reaktionsgemisch einer Destillationskolonne zu, in der man zunächst das überschüssige Thionylchlorid und anschließend, bevorzugt unter Vakuum, das gewünschte organische Chlorid abdestilliert.

Das erfindungsgemäße Verfahren ermöglicht die Herstellung von organischen Chloriden, bei denen das Chloratom an eine CH₂-Gruppe gebunden ist, wobei als Edukte Alkohole, welche im Allgemeinen leicht zugänglich und technisch gut verfügbar sind, sowie leicht zugängliches und technisch gut verfügbares Thionylchlorid und als Katalysator bevorzugt sehr gut verfügbares Triphenylphosphinoxid einzusetzen sind. Ferner führt das gefundene Verfahren auch unter milden Temperaturen und Drücken zu einem hohen Umsatz, einer hohen Selektivität und einer hohen Raum/Zeit-Ausbeute an Wertprodukt und ermöglicht eine einfache Aufarbeitung des Reaktionsgemisches, wobei das Wertprodukt in hoher Reinheit gewonnen werden kann.

### Beispiele

### Beispiel 1 (Vergleichsbeispiel ohne Katalysator)

In einer 250 mL Vierhals-Rührapparatur mit Kühler, Blasenzähler, Tropftrichter, Thermometer und Blattrührer wurden 31,20 g (0,30 Mol) 2,2-Dimethyl-1,3-propandiol (Neopentylglycol) bei Raumtemperatur vorgelegt und im Ölbad auf 80°C aufgeheizt. Auf das feste Edukt wurde nun unter Rühren Thionylchlorid getropft. Die Reaktion sprang sofort an, was sich an der starken Gasentwicklung zeigte. Dabei stieg die Innentemperatur auf 85°C an und das feste 2,2-Dimethyl-1,3-propandiol begann, sich in eine Flüssigkeit umzuwandeln. Nach Zugabe von etwa 44,6 g (0,375 Mol) Thionylchlorid innerhalb einer Stunde wurde trotz weiterem Zutropfen von Thionylchlorid keine weitere Gasentwicklung mehr beobachtet. Die Innentemperatur wurde daraufhin auf 100°C angehoben und weiteres Thionylchlorid bis zu einer Gesamtmenge von 89,25 g (0,75 Mol) und einer Gesamt-Zutropfzeit von etwa 2 Stunden zugetropft, wobei weiterhin keine Gasentwicklung mehr stattfand. Der Ansatz wurde nun bei einer Ölbadtemperatur von 130°C 3 Stunden nachgerührt, wobei das Gemisch bei einer Innentemperatur von etwa 111°C refluxierte. Anschließend wurde der Ansatz auf Raumtemperatur abgekühlt und das Gemisch gaschromatographisch analysiert. Es konnte kein 1,3-Dichlor-2,2-dimethylpropan nachgewiesen werden, sondern hauptsächlich der cyclische Ester aus dem Diol und Thionylchlorid.

### Beispiel 2 (erfindungsgemäß mit Katalysator)

In einer Apparatur wie in Beispiel 1 vorbeschrieben wurden 83,20 g (0,80 Mol) 2,2-Dimethyl-1,3-propandiol (Neopentylglycol) und 4,73 g (0,017 Mol) Triphenylphosphinoxid bei Raumtemperatur vorgelegt und im Ölbad auf 80°C aufgeheizt (molares Verhältnis Triphenylphosphinoxid zur Stoffmenge der zu chlorierenden OH-Gruppen gleich 0,011). Dabei schmolzen beide Stoffe unter Schlierenbildung langsam ineinander. Auf die trübe Schmelze wurde nun unter Rühren Thionylchlorid getropft. Die Reaktion sprang sofort an, was sich an der starken Gasentwicklung zeigte. Bereits nach den ersten wenigen Tropfen Thionylchlorid war das Reaktionsgemisch komplett flüssig. Dabei stieg die Innentemperatur kurzzeitig auf 86°C an. Innerhalb von etwa 2,5 Stunden wurden 100,57 g (0,85 Mol) Thionylchlorid zugetropft, wobei gegen Ende dieser Zeitspanne keine weitere Gasbildung mehr beobachtet wurde. Die Innentemperatur wurde daraufhin ohne weiteres Zutropfen von Thionylchlorid auf 100°C angehoben, wobei ab etwa 95°C eine schwache Gasentwicklung beobachtet werden konnte.

Bei etwa 100°C wurden nun weiteres Thionylchlorid bis zu einer Gesamtmenge von 238,00 g (2,00 Mol) und einer Gesamt-Zutropfzeit von etwa 4,5 Stunden zugetropft. Der Ansatz wurde bei einer Ölbadtemperatur von 130°C und einer Innentemperatur von etwa 115°C 2,5 Stunden nachgerührt, wobei weiterhin Gasentwicklung zu beobachten war. Gegen Ende der Nachrührzeit klang die Gasentwicklung jedoch allmählich ab. Anschließend wurde der Ansatz auf Raumtemperatur abgekühlt. Es wurden 141,41 g Reaktionsaustrag erhalten. Dieser wurde gaschromatographisch analysiert und als Hauptprodukt 1,3-Dichlor-2,2-dimethylpropan nachgewiesen. Der Reaktionsaustrag wurde nun im Vakuum bei 13 hPa abs (13 mbar abs) fraktioniert destilliert. Das Ergebnis ist in Tabelle 1 wiedergegeben. Die drei Fraktionen ergaben eine Gesamtmasse an Dichlor-2,2-dimethylpropan von 112,79 g, was einer Ausbeute von 99,89% entspricht.

**Tabelle 1**

| | Fraktion 1 | Fraktion 2 | Fraktion 3 |
|---|---|---|---|
| Übergangstemperatur [°C] | 55 | 55 | 55 |
| Sumpftemperatur [°C] | 75 | 75 | 75 |
| Masse der Fraktion [g] | 48,70 | 42,02 | 24,07 |
| Reinheit [GC-Flächen-%] | 97,2 | 99,5 | 98,3 |
| Masse an 1,3-Dichlor-2,2-dimethylpropan [g] | 47,33 | 41,80 | 23,66 |

### Beispiel 3 (erfindungsgemäß mit Katalysator)

In einer Apparatur wie in Beispiel 1 vorbeschrieben wurden 50,51 g (0,416 Mol) 1,1,1-Tris(hydroxymethyl)ethan und 3,820 g (0,014 Mol) Triphenylphosphinoxid bei Raumtemperatur vorgelegt und im Ölbad auf 135°C aufgeheizt (molares Verhältnis Triphenylphosphinoxid zur Stoffmenge der zu chlorierenden OH-Gruppen gleich 0,011). Dabei schmolzen beide Stoffe unter Schlierenbildung langsam ineinander. Auf die trübe Schmelze wurde nun unter Rühren 10 mL Thionylchlorid getropft. Die Reaktion sprang sofort an, was sich an der starken Gasentwicklung zeigte. Bereits nach den ersten wenigen Tropfen Thionylchlorid war das Reaktionsgemisch komplett flüssig. Innerhalb von etwa 20 min wurden 101,5 mL (2,838 Mol) Thionylchlorid bei 110 bis 120°C zugetropft. Der Ansatz wurde bei einer Temperatur von 111 °C 90 min nachgerührt. Anschließend wurde der Ansatz auf Raumtemperatur abgekühlt. Es wurden 83,89 g Reaktionsaustrag als klare hellbraune Flüssigkeit erhalten. Diese wurde gaschromatographisch analysiert und als Hauptprodukt 1,3-Dichlor-2-chlormethyl-2-methylpropan nachgewiesen. Der Reaktionsaustrag wurde nun im Vakuum bei 13 hPa abs (13 mbar abs) fraktioniert destilliert. Das Ergebnis ist in Tabelle 2 wiedergegeben. Das Produkt wurde in einer Reinheit von 98% und einer Ausbeute von 85% erhalten.

**Tabelle 2**

| | Fraktion |
|---|---|
| Übergangstemperatur [°C] | 74 |
| Sumpftemperatur [°C] | 76 |
| Masse der Fraktion [g] | 63,2 |
| Reinheit [GC-Flächen-%] | 98 |
| Masse an 1,3-Dichlor-2,2-dimethylpropan [g] | 61,9 |

Beispiel 2 und 3 zeigen, dass durch das erfindungsgemäße Verfahren auch 1,3-Dichlor-2,2-dimethylpropan und 1,3-Dichlor-2-chlormethyl-2-methylpropan als sterisch gehinderte Di- bzw. Trichloride in sehr hoher Ausbeute und Reinheit erhältlich ist.

### Beispiel 4 (erfindungsgemäß mit Katalysator)

In einer Apparatur wie in Beispiel 1 vorbeschrieben wurden 51,54 g (0,423 Mol) 1,6-Hexandiol und 4,101 g (0,014 Mol) Triphenylphosphinoxid bei Raumtemperatur vorgelegt und im Ölbad auf 45°C aufgeheizt (molares Verhältnis Triphenylphosphinoxid zur Stoffmenge der zu chlorierenden OH-Gruppen gleich 0,017). Dabei schmolzen beide Stoffe unter Schlierenbildung langsam ineinander. Innerhalb von etwa 95 min wurden 122,4 g (1,029 Mol) Thionylchlorid bei 70 bis 80 °C zugetropft. Der Ansatz wurde bei einer Temperatur von 60 bis 70°C noch 85 min nachgerührt. Anschließend wurde der Ansatz auf Raumtemperatur abgekühlt. Es wurde eine klare hellbraune Flüssigkeit erhalten. Diese wurde gaschromatographisch analysiert und als Hauptprodukt 1,6-Dichlorhexan nachgewiesen. Der Reaktionsaustrag wurde nun im Vakuum bei 13 hPa abs (13 mbar abs) fraktioniert destilliert. Das Ergebnis ist in Tabelle 3 wiedergegeben. Das Produkt wurde in einer Reinheit von >99% und einer Ausbeute von 87% erhalten.

**Tabelle 3**

| | Fraktion |
|---|---|
| Übergangstemperatur [°C] | 68 |
| Sumpftemperatur [°C] | 69 |
| Masse der Fraktion [g] | 57,5 |
| Reinheit [GC-Flächen-%] | 98 |
| Masse an 1,3-Dichlor-2,2-dimethylpropan [g] | 56,9 |

### Beispiel 5 (Vergleichsbeispiel mit Phosgen als Chlorierungsmittel)

In einer Apparatur wie in Beispiel 1 vorbeschrieben wurden 50 mL 1,6-Dichlorhexan zusammen mit 2 g (0,0072 Mol) Triphenylphosphinoxid vorgelegt und auf 115°C erwärmt. Innerhalb von 75 Minuten wurden 80 g (1,24 Mol) gasförmiges Phosgen und eine Suspension aus 24 g (0,200 Mol) 1,1,1-Tris(hydroxymethyl)ethan in 150 mL 1,6-Dichlorhexan zudosiert (molares Verhältnis Triphenylphosphinoxid zur Stoffmenge der zu chlorierenden OH-Gruppen gleich 0,012). Anschließend wurde für eine weitere Stunde bei 120°C gerührt. Nach Entfernen des nicht-umgesetzten Phosgens durch Einleiten von Stickstoff wurde das erhaltene Produkt gaschromatographisch analysiert. Der Reaktionsaustrag enthielt 2,5 GC-Flächen-% 1,3-Dichlor-2-chlormethyl-2-methyl-propan, 80 GC-Flächen-% Gemische aus chloridsubstituierten Chlorformiaten und 17,5 GC-Flächen-% cyclisches Carbonat.

Das Vergleichsbeispiel 5 zeigt, dass im Gegensatz zum erfindungsgemäßen Beispiel 3 bei der Chlorierung sterisch gehinderter Alkohole mit Phosgen geringe Mengen an Triarylphosphinoxid nicht ausreichend sind und sich somit das Phosgen vom Thionylchlorid im Ergebnis stark unterscheiden.

## Patentansprüche

1. Verfahren zur Herstellung von organischen Chloriden, bei denen das Chloratom an eine CH₂-Gruppe gebunden ist, durch Umsetzung der entsprechenden Alkohole mit Thionylchlorid in Gegenwart eines Triarylphosphinoxids bei einer Temperatur von 20 bis 200°C und einem Druck von 0,01 bis 10 MPa abs, **dadurch gekennzeichnet, dass** man das Triarylphosphinoxid in einem molaren Verhältnis zur Stoffmenge der zu chlorierenden OH-Gruppen von 0,0001 bis 0,5 einsetzt.

2. Verfahren nach Anspruch 1, **dadurch gekennzeichnet, dass** man das Triarylphosphinoxid in einem molaren Verhältnis zur Stoffmenge der zu chlorierenden OH-Gruppen von 0,001 bis 0,1 einsetzt.

3. Verfahren nach den Ansprüchen 1 bis 2, **dadurch gekennzeichnet, dass** man als Alkohol eine Verbindung der allgemeinen Formel (I) 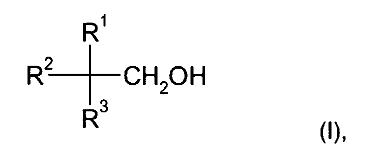 in der die Reste R¹ bis R³ unabhängig voneinander
- für Wasserstoff oder einen Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 30 Kohlenstoffatomen;
- zwei Reste zusammen für einen zweibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 40 Kohlenstoffatomen; oder
- alle drei Reste zusammen für einen dreibindigen, Kohlenstoff enthaltenden organischen, gesättigten oder ungesättigten, acyclischen oder cyclischen, aliphatischen, aromatischen oder araliphatischen, unsubstituierten oder durch 1 bis 5 Heteroatome oder funktionelle Gruppen unterbrochenen oder substituierten Rest mit 1 bis 50 Kohlenstoffatomen
stehen, einsetzt.

4. Verfahren nach Anspruch 3, **dadurch gekennzeichnet, dass** man als Alkohol eine Verbindung der allgemeinen Formel (I), in der die Reste R¹ bis R³ unabhängig voneinander für
- Wasserstoff;
- gegebenenfalls durch Hydroxy, Halogen, Cycloalkyl, Aryl, Aryloxy und/oder Alkyloxy substituiertes und/oder durch ein oder mehrere Sauerstoffatome unterbrochenes C₁- bis C₂₀-Alkyl;
- gegebenenfalls durch Hydroxy, Halogen, Cycloalkyl, Aryl, Aryloxy und/oder Alkyloxy substituiertes und/oder durch ein oder mehrere Sauerstoffatome unterbrochenes C₂- bis C₂₀-Alkenyl, wobei dieses eine oder mehrere C=C-Doppelbindungen enthalten kann;
- gegebenenfalls durch Hydroxy, Halogen, Cycloalkyl, Aryl, Aryloxy und/oder Alkyloxy substituiertes und/oder durch ein oder mehrere Sauerstoffatome unterbrochenes C₂- bis C₂₀-Alkinyl, wobei dieses eine oder mehrere C≡C-Dreifachbindungen enthalten kann; oder
- gegebenenfalls durch Hydroxy, Halogen, Cycloalkyl, Aryl, Alkyl, Aryloxy und/oder Alkyloxy substituiertes C₆- bis C₁₂-Aryl
stehen, einsetzt.

5. Verfahren nach Anspruch 4, **dadurch gekennzeichnet, dass** man als Alkohol eine Verbindung der allgemeinen Formel (I) einsetzt, bei der maximal ein Rest aus R¹ bis R³ Wasserstoff ist.

6. Verfahren nach Anspruch 5, **dadurch gekennzeichnet, dass** man als Alkohol eine Verbindung der allgemeinen Formel (I) einsetzt, bei der kein Rest aus R¹ bis R³ Wasserstoff ist.

7. Verfahren nach Anspruch 6, **dadurch gekennzeichnet, dass** man als Alkohol 2,2-Dimethyl-1,3-propandiol einsetzt.

8. Verfahren nach den Ansprüchen 1 bis 7, **dadurch gekennzeichnet, dass** man als Triarylphosphinoxid Triphenylphosphinoxid einsetzt.

9. Verfahren nach den Ansprüchen 1 bis 8, **dadurch gekennzeichnet, dass** man das Thionylchlorid in einem molaren Verhältnis zur Stoffmenge der zu chlorierenden OH-Gruppen von 0,9 bis 5 einsetzt.

## Claims

1. A process for preparing organic chlorides in which the chlorine atom is bonded to a CH₂ group by reacting the corresponding alcohols with thionyl chloride in the presence of a triarylphosphine oxide at a temperature of from 20 to 200°C and a pressure of from 0.01 to 10 MPa abs, which comprises using the triarylphosphine oxide in a molar ratio to the amount of OH groups to be chlorinated of from 0.0001 to 0.5.

2. The process according to claim 1, wherein the triarylphosphine oxide is used in a molar ratio to the amount of OH groups to be chlorinated of from 0.001 to 0.1.

3. The process according to claims 1 and 2, wherein the alcohol used is a compound of the general formula (I) 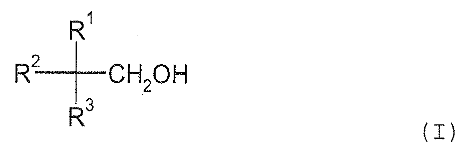 in which the R¹ to R³ radicals are each independently
- hydrogen or a carbon-comprising organic radical which is saturated or unsaturated, acyclic or cyclic, aliphatic, aromatic or araliphatic, unsubstituted, or interrupted or substituted by from 1 to 5 heteroatoms or functional groups and has from 1 to 30 carbon atoms;
- two radicals together are a divalent carbon-comprising organic radical which is saturated or unsaturated, acyclic or cyclic, aliphatic, aromatic or araliphatic, unsubstituted, or interrupted or substituted by from 1 to 5 heteroatoms or functional groups and has from 1 to 40 carbon atoms; or
- all three radicals together are a trivalent carbon-comprising organic radical which is saturated or unsaturated, acyclic or cyclic, aliphatic, aromatic or araliphatic, unsubstituted, or interrupted or substituted by from 1 to 5 heteroatoms or functional groups and has from 1 to 50 carbon atoms.

4. The process according to claim 3, wherein the alcohol used is a compound of the general formula (I) in which the R¹ to R³ radicals are each independently
- hydrogen;
- C₁- to C₂₀-alkyl which is optionally substituted by hydroxyl, halogen, cycloalkyl, aryl, aryloxy and/or alkyloxy and/or interrupted by one or more oxygen atoms;
- C₂- to C₂₀-alkenyl which is optionally substituted by hydroxyl, halogen, cycloalkyl, aryl, aryloxy and/or alkyloxy and/or interrupted by one or more oxygen atoms, and may comprise one or more C=C double bonds;
- C₂- to C₂₀-alkynyl which is optionally substituted by hydroxyl, halogen, cycloalkyl, aryl, aryloxy and/or alkyloxy and/or interrupted by one or more oxygen atoms, and may comprise one or more C=C triple bonds; or
- C₆- to C₁₂-aryl optionally substituted by hydroxyl, halogen, cycloalkyl, aryl, alkyl, aryloxy and/or alkyloxy.

5. The process according to claim 4, wherein the alcohol used is a compound of the general formula (I) in which not more than one radical from R¹ to R³ is hydrogen.

6. The process according to claim 5, wherein the alcohol used is a compound of the general formula (I) in which no radical from R¹ to R³ is hydrogen.

7. The process according to claim 6, wherein the alcohol used is 2,2-dimethyl-1,3-propanediol.

8. The process according to claims 1 to 7, wherein the triarylphosphine oxide used is triphenylphosphine oxide.

9. The process according to claims 1 to 8, wherein the thionyl chloride is used in a molar ratio to the amount of OH groups to be chlorinated of from 0.9 to 5.

## Revendications

1. Procédé de fabrication de chlorures organiques, dans lesquels l'atome de chlore est relié à un groupement CH₂, par mise en réaction des alcools correspondants avec du chlorure de thionyle en présence d'un oxyde de triarylphosphine à une température de 20 à 200 °C et une pression de 0,01 à 10 MPa abs, **caractérisé en ce que** l'oxyde de triarylphosphine est utilisé en un rapport molaire par rapport à la quantité de groupements OH à chlorer de 0,0001 à 0,5.

2. Procédé selon la revendication 1, **caractérisé en ce que** l'oxyde de triarylphosphine est utilisé en un rapport molaire par rapport à la quantité de groupements OH à chlorer de 0,001 à 0,1.

3. Procédé selon les revendications 1 à 2, **caractérisé en ce qu'**un composé de formule générale (I) 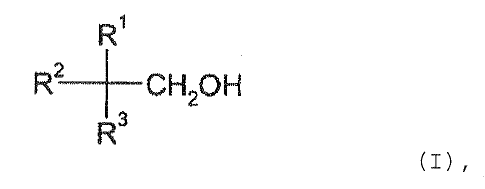 dans lequel, indépendamment les uns des autres,
- les radicaux R¹ à R³ représentent l'hydrogène ou un radical carboné ayant de 1 à 30 atomes de carbone, organique, saturé ou insaturé, acylique ou cyclique, aliphatique, aromatique ou araliphatique, non substitué ou substitué ou interrompu par 1 à 5 hétéroatomes ou groupements fonctionnels ;
- deux radicaux parmi les radicaux R¹ à R³ représentent ensemble un radical carboné à deux liaisons ayant de 1 à 40 atomes de carbone, organique, saturé ou insaturé, acylique ou cyclique, aliphatique, aromatique ou araliphatique, non substitué ou substitué ou interrompu par 1 à 5 hétéroatomes ou groupements fonctionnels ; ou
- les trois radicaux R¹ à R³ représentent ensemble un radical carboné à trois liaisons ayant de 1 à 50 atomes de carbone, organique, saturé ou insaturé, acylique ou cyclique, aliphatique, aromatique ou araliphatique, non substitué ou substitué ou interrompu par 1 à 5 hétéroatomes ou groupements fonctionnels,
est utilisé en tant qu'alcool.

4. Procédé selon la revendication 3, **caractérisé en ce qu'**un composé de formule générale (I) dans lequel, indépendamment les uns des autres, les radicaux R¹ à R³ représentent
- l'hydrogène ;
- un alkyle en C₁ à C₂₀ éventuellement substitué par hydroxy, halogène, cycloalkyle, aryle, aryloxy et/ou alkyloxy et/ou interrompu par un ou plusieurs atomes d'oxygène ;
- un alcényle en C₂ à C₂₀ éventuellement substitué par hydroxy, halogène, cycloalkyle, aryle, aryloxy et/ou alkyloxy et/ou interrompu par un ou plusieurs atomes d'oxygène, ce composé pouvant contenir une ou plusieurs doubles liaisons C=C ;
- un alcynyle en C₂ à C₂₀ éventuellement substitué par hydroxy, halogène, cycloalkyle, aryle, aryloxy et/ou alkyloxy et/ou interrompu par un ou plusieurs atomes d'oxygène, ce composé pouvant contenir une ou plusieurs triples liaisons C≡C ; ou
- un aryle en C₆ à C₁₂ éventuellement substitué par hydroxy, halogène, cycloalkyle, aryle, alkyle, aryloxy et/ou alkyloxy,
est utilisé en tant qu'alcool.

5. Procédé selon la revendication 4, **caractérisé en ce qu'**un composé de formule générale (I) dans lequel au plus un radical parmi R¹ à R³ représente l'hydrogène est utilisé en tant qu'alcool.

6. Procédé selon la revendication 5, **caractérisé en ce qu'**un composé de formule générale (I) dans lequel aucun radical parmi R¹ à R³ ne représente l'hydrogène est utilisé en tant qu'alcool.

7. Procédé selon la revendication 6, **caractérisé en ce que** le 2,2-diméthyl-1,3-propanediol est utilisé en tant qu'alcool.

8. Procédé selon les revendications 1 à 7, **caractérisé en ce que** l'oxyde de triphénylphosphine est utilisé en tant qu'oxyde de triarylphosphine.

9. Procédé selon les revendications 1 à 8, **caractérisé en ce que** le chlorure de thionyle est utilisé en un rapport molaire par rapport à la quantité de groupements OH à chlorer de 0,9 à 5.
